# Europäisches Patentamt
## European Patent Office
## Office européen des brevets

(11) Veröffentlichungsnummer: **0 019 863**
**B1**

(12) **EUROPÄISCHE PATENTSCHRIFT**

(45) Veröffentlichungstag der Patentschrift:
16.06.82

(51) Int. Cl.³: **C 07 C 19/045**, C 07 C 17/156 //
B01J27/10

(21) Anmeldenummer: 80102856.4

(22) Anmeldetag: 22.05.80

(54) **Verfahren zur Herstellung von 1,2-Dichlorethan.**

(30) Priorität: 01.06.79 DE 2922375

(43) Veröffentlichungstag der Anmeldung:
10.12.80 Patentblatt 80/25

(45) Bekanntmachung des Hinweises auf die Patenterteilung:
16.06.82 Patentblatt 82/24

(84) Benannte Vertragsstaaten:
**BE DE FR GB IT NL SE**

(56) Entgegenhaltungen:
**DE-A-2 649 533**
**DE-A-2 733 502**
**FR-A-1 483 132**
**GE-A-1 087 703**
**US-A-3 642 921**

(73) Patentinhaber: **HOECHST AKTIENGESELLSCHAFT,**
**Postfach 80 03 20, D-6230 Frankfurt/Main 80 (DE)**

(72) Erfinder: **Riedl, Josef, Dr., Kantstrasse 53,**
**D-8261 Burgkirchen/Alz (DE)**
Erfinder: **Kühn, Wenzel, Dr., Kampenwandstrasse 15,**
**D-8261 Burgkirchen/Alz (DE)**
Erfinder: **Widmann, Peter, Hüttenbergerweg 37a,**
**D-8262 Altötting (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zur Herstellung von 1,2-Dichlorethan

Die Erfindung betrifft ein Verfahren zur Herstellung von 1,2-Dichlorethan, wobei in einem gemeinsamen Reaktionsraum Ethylen mit Chlorwasserstoff und sauerstoffhaltigem Inertgas einerseits sowie mit Chlor andererseits in der Gasphase in Gegenwart eines aufgewirbelten, festen Katalysators umgesetzt wird.

1,2-Dichlorethan wird bereits seit einer Reihe von Jahren in grossem Masse industriell hergestellt. Es wird hauptsächlich durch thermische Spaltung zu Vinylchlorid umgesetzt, welches wiederum die Grundlage für den Massenkunststoff Polyvinylchlorid ist. Diese Verwendung hat 1,2-Dichlorethan zu einem der am meisten produzierten aliphatischen, chlorierten Kohlenwasserstoffe gemacht. Zu seiner Herstellung sind eine Reihe verschiedener Verfahren bekannt, von denen die meisten von Ethylen ausgehen. Im allgemeinen wird an Ethylen direkt elementares Chlor angelagert, wobei man bei Temperaturen von 40 bis etwa 120°C in flüssiger Phase häufig in 1,2-Dichlorethan arbeitet. In einer viel gebrauchten Form dieses Verfahrens wird die bei der Chloranlagerung entstehende beträchtliche Wärmemenge durch siedendes 1,2-Dichlorethan abgeführt. Da der Siedepunkt des 1,2-Dichlorethans bei normalem Atmosphärendruck bei etwa 84°C liegt, reicht das Temperaturniveau, bei dem die Wärmemenge abgeführt wird, entweder nicht aus, um Wasserdampf zu erzeugen, oder es kann Wasserdampf nur bei niedriger Temperatur und damit niedrigem Druckniveau erhalten werden, der zur Wiederverwendung der in ihm enthaltenen Energie nur beschränkt einsatzfähig ist.

Zur besseren Ausnutzung der Reaktionswärme der direkten Chloranlagerung an Ethylen ist es bekannt, die Umsetzung in der Gasphase in Gegenwart eines aufgewirbelten Katalysators durchzuführen und unmittelbar anschliessend das gebildete 1,2-Dichlorethan zu Vinylchlorid zu spalten. Die Katalysatorteilchen wirken hierbei als Wärmeüberträger, es wird bei Temperaturen von 370 bis 540°C und Drücken bis 2,2 MPa, vorzugsweise bei 0,45 bis 1,85 MPa, gearbeitet. Der bei der Spaltung von 1,2-Dichlorethan entstehende Chlorwasserstoff wird in einem getrennten Apparat für die Oxichlorierung von Ethylen verwendet. Das hieraus gewonnene 1,2-Dichlorethan wird in den Wirbelbett-Spaltreaktor zurückgeleitet.

Nachteile des Verfahrens, wie die Bildung beträchtlicher Mengen Ethylenchlorid, verhältnismässig grosse Anteile an nichtumgesetztem 1,2-Dichlorethan in den Spaltprodukten, Schwierigkeiten bei der Regelung und Steuerung des Prozesses, Tendenz zur Bildung unerwünschter polychlorierter Kohlenwasserstoffe und zur Verharzung und Verkokung des Spaltreaktors, sollen vermindert werden, wenn anstelle von beispielsweise Dehydrochlorierungskatalysatoren fluidisierte, nichtkatalytische Feststoffe in dem Reaktor verwendet werden. Ferner muss das Chlor in der Chlorierungsreaktionszone an einer Anzahl verschiedener Stellen kontrolliert eingeführt werden, um die Verkokungsgefahr herabzusetzen. Hierzu ist ein besonderer, relativ aufwendiger Einbau in den Wirbelbett-Spaltreaktor erforderlich. Es besteht ferner die Schwierigkeit, die heissen Spaltgae vom aufgewirbelten, feinteiligen, festen Wärmeübertragungsmedium möglichst vollständig zu trennen, und der Nachteil, dass bei diesen Verfahren nicht der industriell bevorzugte Röhrenspaltofen für flüssiges oder gasförmiges 1,2-Dichlorethan verwendet werden kann, der hohe Durchsatzleistungen ermöglicht.

Es ist ferner ein Verfahren zur Herstellung von 1,2-Dichlorethan bekannt, wobei in einer ersten Stufe überschüssiges Ethylen, Chlorwasserstoff und überschüssiger Sauerstoff in Form von Luft in Gegenwart eines bekannten Oxichlorierungskatalysators durch entsprechende Einstellung der Mengenverhältnisse der Ausgangsstoffe unter mehr als 90%igem Umsatz an Chlorwasserstoff bei 180 bis 350°C zur Reaktion gebracht werden, die Restgase aus dieser Stufe nach Auswaschen des nichtumgesetzten Chlorwasserstoffs und der dadurch bedingten Kondensation eines Grossteils des entstandenen 1,2-Dichlorethans in einer zweiten Stufe mit 80 bis 120 Mol-% Chlor, bezogen auf das in dieser Stufe eingesetzte Ethylen, in Gegenwart eines eisenhaltigen Katalysators bei 80 bis 250°C umgesetzt werden.

Ein ähnliches Verfahren arbeitet ebenfalls in zwei getrennten Stufen, zwischen denen 1,2-Dichlorethan und Wasser aus dem Reaktionsprodukt abgeschieden werden, wobei in der zweiten Stufe das überschüssige Ethylen aus der Oxichlorierung mit Chlor bei einer Temperatur von 80 bis 320°C in Gegenwart eines aktivierten Aluminiumoxid-Katalysators umgesetzt wird.

Neuerdings wurde bekannt, die bei der Nachchlorierung des überschüssigen Ethylens aus der Oxichlorierung gebildeten erheblichen Mengen an 2-Chlorethanol dadurch herabzusetzen, dass man die Umsetzung in Gegenwart von zugesetztem Chlorwasserstoff durchführt.

Schliesslich ist ein Verfahren bekannt, die Nebenprodukte bei der Chlorierung von ethylenhaltigen Gasen, die nach der Oxichlorierung und Abtrennung der Hauptmenge der gebildeten organischen Produkte durch Abschrecken mit Wasser anfallen, dadurch zu vermindern, dass man die Chlorierung in Gegenwart von Kupfer(II)-chlorid und/oder Eisen(III)-chlorid auf einem Träger als Katalysator durchführt.

Alle diese letztgenannten Verfahren haben den Nachteil, dass ein zusätzlicher Reaktor mit Produktabscheider und weiteren Einrichtungen benötigt wird, um die Ausbeute an 1,2-Dichlorethan, bezogen auf das eingesetzte Ethylen, bei der Oxichlorierung zu verbessern. Es wurde nun ein Verfahren gefunden, mit dem man sowohl die Oxichlorierung wie auch die direkte Chloranlagerung an Ethylen in einem gemeinsamen Reaktionsraum mit guten Ausbeuten an 1,2-Dichlorethan ausführen kann, wobei das Temperaturniveau der Reaktion eine wesentlich bessere Ausnutzung der abgeführten Reaktionswärme der Chloranlagerung an Ethylen ermöglicht als nach dem bisher viel verwendeten Verfahren der Chlorierung in siedendem 1,2-Dichlorethan.

Das neue Verfahren zur Herstellung von 1,2-Dichlorethan aus Ethylen durch Umsetzung mit Chlorwasserstoff· und Sauerstoff enthaltenden Inertgasen bei 180 bis 300°C und 0,09 bis 1,1 MPa sowie durch Umsetzung mit Chlor in der Gasphase in Gegenwart eines Kupfersalz oder Kupfer- und Eisensalz enthaltenden festen Katalysators mit anschliessender Abkühlung und destillativer Auftrennung des Reaktionsgemisches ist dadurch gekennzeichnet, dass beide Chlorierungsreaktionen in einem gemeinsamen Reaktionsraum, der aufgewirbelte Katalysatorteilchen enthält, nacheinander durchgeführt werden, wobei für die erste Chlorierungsreaktion ein stöchiometrischer Überschuss von Ethylen, bezogen auf das Chlorierungsmittel, eingesetzt wird, und die im gesamten Reaktionsraum gebildete Wärme durch indirekte Kühlung mit einem flüssigen und/oder gasförmigen Wärmeübertragungsmedium abgeführt wird.

Die beiden Chlorierungsreaktionen können in beliebiger Reihenfolge nacheinander durchgeführt werden, wobei ein qualitativ hochwertiges 1,2-Dichlorethan in guten Ausbeuten auch dann erhalten wird, wenn die umgesetzte Menge Ethylen bei jeder Chlorierungsreaktion etwa gleich gross ist. Letzteres Verfahren ist eine bevorzugte Variante des erfindungsgemässen Verfahrens, wenn das erzeugte 1,2-Dichlorethan anschliessend wie üblich durch thermische Spaltung zu Vinylchlorid umgesetzt wird, da auf diese Weise die Gesamtmenge des zu spaltenden 1,2-Dichlorethans in einer Reaktionseinheit unter guter Wärmeausnutzung erzeugt werden kann, wobei der durch den Spaltprozess erzeugte Chlorwasserstoff in die 1,2-Dichlorethan-Herstellung zurückgeführt und zur Chlorierung von etwa der Hälfte des insgesamt eingesetzten Ethylens verwendet wird.

Als Wärmeübertragungsmedium für die Abführung der im gesamten Reaktionsraum gebildeten Wärme durch indirekte Kühlung eignen sich beispielsweise hochsiedende Mineralöle und Silikonöle. Bevorzugt wird Wasser eingesetzt, das durch Wärmeaufnahme in Mitteldruckdampf verwandelt wird.

In einer bevorzugten Ausführungsform des erfindungsgemässen Verfahrens wird in den Reaktionsraum zuerst Chlorwasserstoff, Ethylen und Sauerstoff enthaltendes Inertgas und danach Chlor in folgenden Molverhältnissen eingeführt:

2 Mol HCl; 1,01 bis 3 Mol $C_2H_4$; mindestens 0,5, im allgemeinen 0,5 bis 1 Mol $O_2$ und 0,009 bis 2 Mol $Cl_2$, wobei die Chlormenge so bemessen wird, dass im Endprodukt der Reaktion, das heisst im Gasgemisch, das den Reaktionsraum verlässt, weniger als 0,001 Gew.-% freies, elementares Chlor gefunden werden.

Unter Inertgas sind solche Stoffe zu verstehen, die unter den Reaktionsbedingungen gasförmig sind und an der Reaktion entweder überhaupt nicht oder in ganz untergeordnetem Masse teilnehmen. Beispiele für Inertgase sind Stickstoff, Kohlendioxid und 1,2-Dichlorethan-Dampf. Bevorzugt wird Stickstoff als Inertgas eingesetzt. Die Inertgas-Menge wird zweckmässig so bemessen, dass eine ausreichende Aufwirbelung der festen Katalysatorteilchen erreicht wird, ohne das Reaktionsgemisch zu weitgehend zu verdünnen.

Bevorzugt wird der Sauerstoff zu mindestens 50 bis 100%, insbesondere zu 90 bis 100%, seiner Gesamtmenge als Luft in den Reaktionsraum eingeleitet.

Alle Gase werden möglichst mit geringer relativer Feuchte in die Reaktionszone gegeben. Das Chlorwasserstoffgas stammt bevorzugt aus der thermischen Spaltung von 1,2-Dichlorethan zur Herstellung von Vinylchlorid. Vor Eingabe in den Reaktionsraum können die Gase beispielsweise auf Temperaturen von 60 bis 180°C vorgeheizt werden.

Alle Gase können einzeln in den Reaktionsraum eingeführt werden, bevorzugt werden jedoch Chlorwasserstoff und Ethylen einerseits sowie Sauerstoff und Inertgas, beispielsweise als Luft, andererseits, jeweils in Mischung miteinander, eingeführt. Das Chlor kann bei diskontinuierlicher Fahrweise zeitlich, bei der bevorzugten kontinuierlichen Fahrweise räumlich nach der Eingabe der anderen Gase in den Reaktionsraum eingeführt werden.

Der Reaktionsraum kann beispielsweise kugelige, ellipsoidische oder zylindrische Gestalt haben, er sollte so ausgebildet sein, dass er keine toten Ecken und Winkel aufweist, in denen sich der aufgewirbelte Katalysator absetzen kann. Vorzugsweise wird ein langgestreckt zylindrischer Reaktionsraum mit kreisförmigem Querschnitt und senkrecht stehender Zylinderachse, beispielsweise ein Rohr, verwendet.

Der Reaktionsraum ist zweckmässig mit einem Doppelmantel sowie Einbauten ausgerüstet, durch die das Wärmeübertragungsmedium fliesst. Geeignete Einbauten sind beispielsweise Schlangen- oder Rohrregister-Kühler. Diese Einbauten können in mehreren, voneinander getrennten Einheiten angeordnet sein, die von verschiedenen Medien mit unterschiedlichen Strömungsgeschwindigkeiten durchflossen werden können, um eine optimale Wärmeausnutzung und einen optimalen Temperaturverlauf im Reaktionsraum zu ermöglichen.

Die verschiedenen Gase können durch einfache Rohre in den Reaktionsraum geleitet werden, die zweckmässig an ihren Enden Einrichtungen zur besseren Verteilung über die Fläche enthalten. Solche geeigneten Einrichtungen sind beispielsweise gelochte Platten oder Kugeln, Fritten oder ein bzw. mehrere Rohre mit einer Vielzahl von Gasausström-Öffnungen.

Der Reaktionsraum enthält zweckmässig in seiner obersten Zone eine Öffnung mit regelbarem Querschnitt, durch die die Reaktionsprodukte abgeführt werden. Nach Verlassen des Reaktionsraumes passieren die Reaktionsprodukte zweckmässig einen Abscheider für feinteilige, feste Katalysatorteilchen, beispielsweise einen Zyklon oder ähnlichen Apparat. Die abgeschieden Teilchen werden in den Reaktor zurückgeführt.

Nach Verlassen des Abscheiders werden die Gase gegebenenfalls gewaschen und teilweise kondensiert, die bei Normaldruck bei ca. 10°C nicht kondensierbaren Gasanteile werden, gegebenenfalls nach Abtrennung schädlicher oder störender Stoffe, in die Atmosphäre geleitet. Zumindest ein Teil der nicht kondensierbaren Gase kann auch im Kreis in den Reaktionsraum zurückgeführt werden. Die kondensierten Reaktionsprodukte werden zwecks Gewinnung von reinem 1,2-Dichlorethan destillativ aufgetrennt, wie üblich.

In einer besonders bevorzugten Ausführungsform der vorliegenden Erfindung werden die umzusetzenden Stoffe in den Reaktionsraum in folgenden Mengenverhältnissen eingeführt:

2 Mol HCl; 1,8 bis 2,2 Mol $C_2H_4$; 0,5 bis 0,6 Mol $O_2$ und 0,79 bis 1,2 Mol $Cl_2$. Bei Anwendung dieser Molverhältnisse kann 1,2-Dichlorethan für die nachfolgende Spaltung zu Vinylchlorid unter optimaler Ausnutzung des aus dem Spaltprozess zurückgeführten Chlorwasserstoffs sowie der Einsatzstoffe Ethylen und Chlor in einer einzigen Reaktionseinheit hergestellt werden.

Das im vorangegangenen Abschnitt beschriebene Verfahren wird insbesondere so ausgeführt, dass an einem Ende eines rohrförmigen Reaktors, zweckmässig am unteren Ende eines senkrecht oder nahezu senkrecht stehenden rohrförmigen Reaktors Ethylen, Chlorwasserstoff und sauerstoffhaltiges Inertgas getrennt oder zumindest teilweise getrennt voneinander eingeleitet werden. Es können beispielsweise Ethylen und Chlorwasserstoff gemeinsam, jedoch getrennt vom sauerstoffhaltigen Inertgas eingeführt werden. In einem bestimmten Abstand von der in Gasströmungsrichtung letzten der obengenannten Gaseinleitungen entfernt wird Chlor in den Reaktionsraum eingeleitet. Die Lage der Chloreinleitung wird so gewählt, dass zwischen ihr und der vorhergehenden Gaseinleitung ein Reaktionsraum liegt, der 40 bis 85%, vorzugsweise 55 bis 75% des gesamten zur Verfügung stehenden Reaktionsraumes im Reaktor beträgt. Am anderen Ende des Reaktors, zweckmässig am oberen Ende eines senkrechten oder nahezu senkrecht stehenden rohrförmigen Reaktors werden die Reaktionsprodukte abgeführt.

Ein solches Verfahren ist insbesondere für den technisch wichtigen kontinuierlichen Betrieb geeignet.

Für diesen kontinuierlichen Betrieb mit in Strömungsrichtung der Gase zuerst eingeleitetem Chlorwasserstoff und nachfolgend eingeleitetem Chlor wird vorzugsweise das Wärmeübertragungsmedium in der indirekten Kühlung des Reaktionsraums im Gegenstrom zu den im Reaktionsraum befindlichen Gasen geführt. Hierdurch wird eine bessere Wärmeabführung und ein günstigerer Temperaturverlauf im Reaktionsraum erreicht.

Nach einer weiteren bevorzugten Ausführungsform des erfindungsgemässen Verfahrens wird in den Reaktionsraum zuerst Ethylen, Chlor und Inertgas, das gegebenenfalls Sauerstoff enthalten kann, und nachfolgend Chlorwasserstoff und gegebenenfalls Sauerstoff und Inertgas in folgenden Molverhältnissen eingeführt:

2 Mol $C_2H_4$; 0,9 bis 1,2 Mol $Cl_2$; 1,6 bis 2,3 Mol HCl und 0,35 bis 1,3 Mol Gesamt-$O_2$, wobei die Sauerstoff- oder die Chlorwasserstoffmenge so bemessen wird, dass im Endprodukt, das heisst im Gasgemisch, das den Reaktionsraum verlässt, weniger als 0,001 Gew.-% freies, elementares Chlor gefunden werden. Diese Ausführungsform des Verfahrens wird insbesondere angewendet, wenn, beispielsweise zur Verminderung des Anteils an 2-Chlorethanol in den Reaktionsprodukten, mit einem geringen Überschuss an Chlorwasserstoff gefahren werden soll. Wie bereits oben beschrieben, ist auch diese Ausführungsform geeignet, 1,2-Dichlorethan für die thermische Spaltung zu Vinylchlorid zu erzeugen, unter optimaler Ausnutzung des bei dieser Spaltung erzeugten Chlorwasserstoffs, wobei die gesamte 1,2-Dichlorethan-Erzeugung in einem einzigen Reaktionsraum stattfindet.

Als Inertgas sind wiederum, wie oben beschrieben, beispielsweise Stickstoff, Kohlendioxid und/oder 1,2-Dichlorethan-Dampf geeignet, wobei bevorzugt Stickstoff eingesetzt wird. Die Hauptmenge des benötigten Sauerstoffs wird zweckmässig an der Stelle zugeführt, an der auch der Chlorwasserstoff eingeleitet wird, jedoch ist auch die Zuführung beträchtlicher Mengen Sauerstoff bereits an der Stelle, an der Ethylen und Chlor eingeleitet werden, möglich. Diese Verfahrensweise wird insbesondere dann angewendet, wenn als kostengünstiges Wirbelgas Luft eingesetzt werden soll.

Das in den beiden vorangeganenen Abschnitten beschriebene Verfahren wird insbesondere so ausgeführt, dass an einem Ende eines rohrförmigen Reaktors, zweckmässig am unteren Ende eines senkrecht oder nahezu senkrecht stehenden rohrförmigen Reaktors Ethylen, Chlor und Inertgas, das gegebenenfalls Sauerstoff enthalten kann, zumindest teilweise getrennt voneinander eingeleitet werden. Das Inertgas kann beispielsweise auch in Mischung mit Chlor, jedoch das Ethylen getrennt hiervon eingeführt werden. In einem bestimmten Abstand von der in Gasströmungsrichtung letzten der obengenannten Gaseinleitungen entfernt wird Chlorwasserstoff und gegebenenfalls Sauerstoff und Inertgas getrennt oder zumindest teilweise getrennt in den Reaktionsraum eingeleitet. Die Lage der Chlorwasserstoff-Einleitung wird so gewählt, dass zwischen ihr und der vorhergehenden Gaseinleitung ein Reaktionsraum liegt, der 10 bis 40%, vorzugsweise 15 bis 30%, des gesamten zur Verfügung stehenden Reaktionsraumes im Reaktor beträgt. Am anderen Ende des Reaktors, zweckmässig am oberen Ende eines senkrechten oder nahezu senkrecht stehenden rohrförmigen Reaktors werden die Reaktionsprodukte abgeführt.

Bei der soeben beschriebenen Ausführungsform des neuen Verfahrens wird das Wärmeübertragungsmedium in der indirekten Kühlung des Reaktionsraums vorteilhaft im Gleichstrom zu den Gasen geführt.

Das erfindungsgemässe Verfahren wird vorteilhaft bei Temperaturen des Reaktionsgemisches im Reaktionsraum von 190 bis 250°C, insbesondere bei 200 bis 230°C, durchgeführt. Hierbei kann, insbesondere bei kontinuierlichen Verfahren ein räumlicher Temperaturgradient angewendet werden. Beispielsweise kann an der Einführungsstelle der Gase in den Reaktionsraum eine niedrigere Temperatur herrschen als an der Entnahmestelle der Reaktionsprodukte. Die Temperatur kann auch in Strömungsrichtung der Gase gesehen im ersten Drittel des Reaktionsraumes oder in der Mitte oder im zweiten Drittel höher sein als in den restlichen Bereichen des Reaktionsraumes.

Zweckmässig werden die Gase vor Einführung in den Reaktionsraum auf Temperaturen von 50 bis etwa 180°C erwärmt.

Das neue Verfahren kann bei normalem Atmosphärendruck (0,09 bis 0,1 MPa) durchgeführt werden. Im allgemeinen wird man zur Erhöhung der Raum-Zeit-Ausbeute erhöhten Druck bis etwa 1,1 MPa anwenden. Vorzugsweise wird bei Drücken von 0,3 bis 0,6 MPa gearbeitet.

Der feste Katalysator wird vorteilhaft in feinteiliger Form mit einer mittleren Teilchengrösse von 20 bis 400 µm angewendet. Besonders gute Ergebnisse werden mit einem Katalysator mit einer mittleren Teilchengrösse von 30 bis 70 µm erhalten.

Der Katalysator besteht zweckmässig aus einer Trägersubstanz, die eine grosse Oberfläche je Gewichtseinheit, beispielsweise 70 bis 200 $m^2$/g und mehr, aufweist, bei hohen Temperaturen, beispielsweise bis mindestens 500°C, mechanisch stabil ist und aus der Gasreaktion unverändert hervorgeht. Geeignete Trägermaterialien sind wärmebeständige Oxide, beispielsweise Siliciumdioxid oder Aluminiumoxid sowie Diatomeenerde oder silikatische Materialien. Vorzugsweise wird Aluminiumoxid eingesetzt.

Auf diesem Trägermaterial ist zweckmässig etwa 0,5 bis 15 Gew.-%, bezogen auf den gesamten Katalysator, Kupfer als Salz oder Oxid aufgebracht. Diese Kupfersalze bzw. -oxide verwandeln sich in der Regel während des Gebrauchs durch den anwesenden Chlorwasserstoff und das Chlor in Kupfer(II)-chlorid sofern sie nicht schon von Anfang an als dieses Chlorid aufgebracht wurden.

Neben Kupfer kann der Katalysator mit Vorteil noch kleinere Mengen von Lewis-Säuren, insbesondere etwa 0,01 bis 0,5 Gew.-%, bezogen auf den gesamten Katalysator, Eisen, das als Oxid oder als Salz aufgebracht wurde und sich während der Reaktion in die Lewis-Säure Eisen-(III)-chlorid umwandelt, enthalten. Die obengenannten Prozentangaben beziehen sich jeweils auf das Metallion, nicht auf das Chlorid bzw. sonstige Metallsalz- oder -oxid.

Neben den genannten Zusätzen kann der Katalysator noch weitere Zusätze enthalten, die die Flüchtigkeit insbesondere des Kupfer(II)-chlorids herabsetzen, beispielsweise Alkalimetallchloride wie Kaliumchlorid oder Erdalkalimetallchloride wie Calcium- oder Magnesiumchlorid sowie Zusätze weiterer metallischer Verbindungen, die die Wirksamkeit und/oder Selektivität des Katalysators in bezug auf die Gewinnung von 1,2-Dichlorethan verbessern. Als Beispiele seien genannt Silber, Zink, Chrom, Mangan, seltene Erdmetalle wie Cer, Lanthan, Ytterbium und Neodym; Platinmetalle wie Rhodium, Platin.

Es können auch Mischungen verschiedener Katalysator- und Katalysatorträger-Teilchen verwendet werden, zum Beispiel mit Kupfersalzen behandeltes Trägermaterial, gemischt mit Teilchen des unbehandelten oder eines anders, beispielsweise mit Eisen(III)-chlorid oder einer anderen Lewis-Säure behandelten Trägermaterials.

Das Verhältnis des Gesamtreaktionsraumes vor Einfüllen des Katalysators zum Schüttvolumen des eingefüllten Katalysators beträgt zweckmässig etwa 1,1 bis 3, vorzugsweise 1,2 bis 1,7.

Die Strömungsgeschwindigkeit der Gase im Reaktionsraum ist zweckmässig so hoch, dass mindestens 95 Gew.-%, vorzugsweise 100 Gew.-%, Katalysatorteilchen aufgewirbelt werden. Entsprechend ist die Einspeisung des gegebenenfalls im Kreis geführten Inertgases unter Berücksichtigung der in den Reaktionsraum eingespeisten, an der Reaktion beteiligten Gase zu wählen.

Die mittlere Verweilzeit der in Reaktion befindlichen Gase im Reaktionsraum hängt von der gewählten Reaktionstemperatur ab, wobei im allgemeinen die Verweilzeit um so kürzer sein soll, je höher die Reaktionstemperatur eingestellt wird. Die mittlere Verweilzeit beträgt im allgemeinen 10 bis 100, vorzugsweise 20 bis 70 s, und insbesondere 30 bis 60 s. Sie wird beim kontinuierlichen Betrieb ermittelt aus dem in den Reaktionsraum in einer Sekunde eingespeisten Volumen der Gase bei dem Druck und der Temperatur, die im Reaktionsraum herrschen, in bezug auf das Volumen des gesamten Reaktionsraumes, abzüglich des Eigenvolumens des darin enthaltenen Katalysators und der Einbauten (Kühlrohre; Temperatur-Messfühler). Das Eigenvolumen der Katalysatorteilchen wird beispielsweise nach der Flüssigkeitsverdrängungs-Methode (siehe weiter unten) ermittelt.

Bevorzugt wird bei dem erfindungsgemässen Verfahren soviel Sauerstoff bzw. Sauerstoff enthaltendes Inertgas in den Reaktionsraum eingeführt, dass im Gasgemisch, das den Reaktionsraum verlässt, nach Kondensation der leicht kondensierbaren Reaktionsprodukte (z.B. Wasser und 1,2-Dichlorethan) bei +10°C und Abtrennung des Chlorwasserstoffs durch übliche Wäsche noch 2 bis 9, insbesondere 4 bis 7 Vol.-% Sauerstoff enthalten sind. Es ist beispielsweise im Hinblick auf eine Wäsche der Abgase mit organischen, brennbaren Lösungsmitteln zur Abtrennung von Restmengen an 1,2-Dichlorethan von Vorteil, wenn der $O_2$-Gehalt des wie soeben beschrieben vorbehandelten Abgases nicht zu hoch, beispielsweise unter 9 Vol.-%, liegt. Ist eine solche Nachreinigung nicht vorgesehen, kann der Sauerstoffgehalt auch noch höher, beispielsweise bei 10 bis 13 Vol.-% liegen, wobei immer noch sehr gute Ausbeuten an 1,2-Dichlorethan erzielbar sind.

Die Auftrennung und Reinigung des den Reaktionsraum verlassenden Gasgemisches erfolgt, wie weiter oben bereits beschrieben, nach bekannten Verfahren.

Wie gleichfalls oben bereits angedeutet, ermöglicht es das erfindungsgemässe Verfahren einerseits in einer einzigen Reaktionseinheit Ethylen mit besonders guter Ausbeute durch Oxichlorierung der Hauptmenge und Nachchlorierung der Restmenge des Ethylens zu 1,2-Dichlorethan von guter Qualität umzusetzen. Andererseits ermöglicht das neue Verfahren, durch Verwendung nur einer Reaktionseinheit die Gesamtmenge 1,2-Dichlorethan in guter Qualität und Ausbeute für die weitere thermische Zersetzung zur Herstellung von Vinylchlorid bereitzustellen, unter weitgehend vollständiger Wiederverwendung des bei der thermischen Zersetzung anfallenden Chlorwasserstoffs. In der Reaktionseinheit entsteht eine beträchtliche Wärmemenge auf einem Temperaturniveau, das eine günstige Weiterverwendung dieser Wärme, beispielsweise als Mitteldruckdampf, ermöglicht. Das Verfahren benötigt keine komplizierten, kostenintensiven oder störanfälligen apparativen Einrichtungen, es kann in Apparaten durchgeführt werden, die leicht zu reinigen und instandzuhalten sind.

Nachfolgende Beispiele sollen die Erfindung näher erläutern:

Beispiel 1

Es wird folgende Apparatur verwendet: Zur Ausführung der Umsetzung von Ethylen zu 1,2-Dichlorethan dient ein senkrecht stehendes Glasrohr mit 80 mm innerem Durchmesser, das unten und oben zu je einer Gaseinström- bzw. Gasausströmöffnung verjüngt ist. Dieses senkrecht stehende Reaktionsrohr enthält unmittelbar über der unteren Einströmöffnung eine Glasfritte, die über den gesamten inneren Querschnitt des Reaktionsrohrs verläuft. In kurzem Abstand über dieser ersten Fritte ist eine zweite Fritte angebracht, deren Fläche etwa den halben Querschnitt des Reaktionsrohrs ausmacht und die in ihrem unteren Teil mit einem Glasrohr verbunden ist, das seitlich durch den Mantel des Reaktionsrohres geführt ist. Zur Temperierung enthält das Reaktionsrohr eine Glasrohrschlange, deren Anschlüsse ebenfalls seitlich durch den Mantel des Reaktionsrohres geführt sind und die kurz oberhalb der zweiten Fritte beginnt und im Reaktionrohr soweit nach oben reicht, dass etwa 1/10 der gesamten Länge des Reaktionsrohres im oberen Teil freibleiben. Zwischen der zweiten Fritte und dem oberen Ende des Reaktionsrohres sind über den Mantel des Rohres gleichmässig verteilt 6 Stutzen angebracht, durch die Temperatur-Messfühler in das Innere des Reaktionsrohres reichen. In bestimmten Abständen oberhalb der Fritte 2 enthält der Mantel des Reaktionsrohres zwei weitere Stutzen, durch die Gaseinleitungsrohre geführt werden können, die bis in die Mitte des Reaktionsrohres reichen, dort senkrecht nach unten gebogen sind und in einer gelochten Kugel enden. Sofern das Gaseinleitungsrohr durch den Stutzen geführt ist, der in grösserer Entfernung von der zweiten Fritte angebracht ist, beträgt der Abstand zwischen der gelochten Kugel und der zweiten Fritte 69% der gesamten inneren Länge des Reaktionsraumes in dem Reaktionsrohr. Der Reaktionsraum wird gemessen von der Oberfläche der ersten Fritte bis zur Verjüngungsstelle im obersten Teil des Reaktionsrohres. Sofern das Gaseinleitugsrohr durch den Stutzen geführt ist, der näher an der zweiten Fritte liegt, beträgt der Abstand von der gelochten Kugel des Gaseinleitungsrohrs bis zur zweiten Fritte 17% der gesamten Länge des Reaktionsraumes im Reaktionsrohr. Der gesamte Mantel des Reaktionsrohres ist mit einer Wärmeisolierschicht versehen.

Oberhalb des Reaktionsrohres ist eine Glaskugel angebracht zwecks Abscheidung von Katalysatorteilchen, die vom Gasstrom mitgerissen werden. Diese Glaskugel wiederum ist über eine absteigende Leitung mit einem Wasserkühler verbunden, an dessen unterem Ende ein Kondensatsammelgefäss mit Ablasshahn angebracht ist. Das Kondensatsammelgefäss enthält in seinem Oberteil ein Gasabführungsrohr, das wiederum in einen aufsteigenden Solekühler mündet. Die hier kondensierten Bestandteile des Gases fliessen in ein zweites Kondensatsammelgefäss mit Ablasshahn. Die den oberen Teil des Solekühlers verlassenden nichtkondensierbaren Abgase werden durch Waschflaschen geleitet, zur Abscheidung des darin enthaltenen Chlorwasserstoffs. Von dem gewaschenen Abgas werden Proben für eine gaschromatographische Analyse entnommen. Die Kondensate, die sich in den beiden unterhalb der Kühler angebrachten Gefässen sammeln, werden vereinigt und ebenfalls gaschromatographisch analysiert.

Die Glaskugel, in der die mitgerissenen Katalysatorteilchen abgeschieden werden, sowie das von ihr ausgehende Überleitungsrohr zum Wasserkühler sind mit elektrischen Heizmanschetten versehen. Diese Apparateteile werden während des Betriebes des Reaktors soweit geheizt, dass hier keine Kondensatbildung auftritt.

Das Volumen des Reaktionsraumes im Reaktionsrohr abzüglich der darin enthaltenen Einbauten (Temperierschlange, zweite Fritte, Gaseinleitungskugel sowie Temperaturmessfühler) beträgt 4700 cm³.

Zur Durchführung des ersten Beispiels wird das Reaktionsrohr mit 2,8 l (Schüttvolumen) eines Katalysators gefüllt, der aus einem Aluminiumoxidträger besteht und 3,7 Gew.-%, bezogen auf den Katalysator, Kupfer in Form eines Salzes und Spuren Eisen enthält. Der Katalysator hat folgende Siebanalyse:

| | |
|---|---|
| Teilchen < 20 μ | 25 Gew.-% |
| Teilchen > 20 μ, jedoch < 70 μ | 65 Gew.-% |
| Teilchen > 70 μ | 10 Gew.-%. |

Das Eigenvolumen des Katalysators wird nach der Wasserverdrängungsmethode bestimmt: 1 Messzylinder mit 2 l Inhalt wird zunächst mit 1 l Katalysatorteilchen gefüllt und dazu 1 l Wasser von 20°C gegeben. Die Mischung wird etwas geschüttelt und einige Zeit stehengelassen bis keine Gasblasen mehr aufsteigen. Das Volumen der Mischung beträgt nun 1300 cm³. 1 l (Schüttvolumen) des Katalysators besitzt demnach ein Eigenvolumen der Katalysatorteilchen von 300 cm³. Die gesamte Katalysatorfüllung von 2,8 l hat ein Eigenvolumen von 840 cm³. Der freie Gasraum im Reaktionsrohr beträgt nach Einfüllung des Katalysators noch 3,86 l.

Nun wird über das untere Gaseinleitungsrohr durch die erste Fritte Luft in einer Menge von 60 Normalliter pro Stunde geblasen und die Temperierschlange im Reaktionsrohr mit einer Heizflüssigkeit geheizt. Nach etwa 25 Minuten wird im Reaktionsrohr eine Lufttemperatur von 185°C gemessen, die sich innerhalb der nächsten 5 Minuten nicht mehr ändert. Nun wird die eingeblasene Luftmenge auf 90 Nl · h⁻¹ erhöht und gleichzeitig über die zweite Fritte ein Gemisch von 45 Nl · h⁻¹ Ethylen und 44 Nl · h⁻¹ Chlorwasserstoffgas eingeleitet. Unmittelbar danach wird auch mit der Einleitung von 22 Nl · h⁻¹ Chlorgas über das mit der Kugel versehene Gaseinleitungsrohr, das in dem von der zweiten Fritte entfernteren Stutzen angebracht ist, begonnen (der nähere Stutzen wird nicht benützt, er ist mit einem Stopfen verschlossen). Alle dem Reaktionsrohr zugeführten Gase sind auf 60°C vorgeheizt.

Zusammen mit der Einleitung der Reaktionsgase wird der Wasserkühler mit Wasser von +13°C und der Solekühler mit Kühlsole von −15°C beschickt. Die Abgaswaschflaschen enthalten Wasser als Waschflüssigkeit.

Nach kurzer Zeit ist die Temperatur im Reaktionsrohr auf 220°C gestiegen. Sie wird im weiteren Verlauf des Versuches durch Beschickung der Temperierschlange mit Kühlflüssigkeit auf dieser Temperatur gehalten. Das den Solekühler verlassende Abgas hat eine Temperatur von +10°C.

Der Versuch wird während 4 Stunden fortgeführt und nach 1/3 und 2/3 dieser Zeit je einmal die Abgaszusammensetzung des gewaschenen Abgases gaschromatographisch ermittelt. Für die Gase Sauerstoff, Kohlenoxid, Kohlendioxid und Ethylen wird ein Wärmeleitfähigkeitsdetektor für alle anderen nachstehend angegebenen Gase ein Flammionisationsdetektor verwendet. Die Mittelwerte aus beiden Analysen sind für alle Beispiele in der weiter unten stehenden Tabelle II zusammengefasst, wobei vom Sauerstoffwert der über die verwendete Luft mitgebrachte Edelgasanteil bereits abgerechnet ist.

Nach Ablauf der Versuchsdauer wird die Gaszufuhr zum Reaktionsrohr beendet und der Katalysator mit Luft (etwa Zimmertemperatur) kaltgeblasen. Das vom Wasser und Solekühler gebildete Kondensat wird vereinigt, gewogen und ebenfalls gaschromatographisch mit einem Flammionisationsdetektor analysiert. Die für die einzelnen Beispiele ermittelten Werte sind in der weiter unten stehenden Tabelle I aufgeführt.

Für den Versuch gemäss Beispiel 1 werden folgende Werte errechnet:

Molverhältnis HCl : $C_2H_4$ : $Cl_2$ : $O_2$ = 2 : 2,05 : 1 : 0,86.

Umsatz, bezogen auf: HCl = 96%; $Cl_2$ = 99,99%; $C_2H_4$ = 97,5%.

Raum-Zeit-Ausbeute (bezogen auf 3,86 l Reaktionsraum): 49,2 g Roh-1,2-Dichlorethan · h⁻¹ · l⁻¹.

Mittlere Verweilzeit der Gase im Reaktionsraum: 36,8 s.

Beispiel 2

Es wird dieselbe Apparatur verwendet wie in Beispiel 1 und genauso verfahren, wie dort angegeben, mit dem Unterschied, dass die Temperatur im Reaktionsrohr auf 200°C während einer Versuchsdauer von insgesamt 5 Stunden gehalten wird.

Folgende Werte werden ermittelt:

Molverhältnis: HCl : $C_2H_4$ : $Cl_2$ : $O_2$ = 2 : 2,05 : 1 : 0,86.

Umsatz, bezogen auf: HCl = 92%, $Cl_2$ = 99,9%; $C_2H_4$ = 96%.

Raum-Zeit-Ausbeute (bezogen auf 3,86 l Reaktionsraum): 48,3 g Roh-1,2-Dichlorethan · h⁻¹ · l⁻¹.

Mittlere Verweilzeit der Gase im Reaktionsraum: 38,3 s.

Analysen siehe Tabellen I und II.

Beispeil 3

Wiederum wird verfahren wie in Beispiel 1 bzw. Beispiel 2, mit dem Unterschied, dass die Temperatur im Reaktionsrohr auf 240°C konstant gehalten wird. Die Versuchsdauer beträgt 4½ Stunden.

Folgende Werte werden ermittelt:

Umsatz bezogen auf: HCl = 98%; $Cl_2$ = 100%; $C_2H_4$ = 98%.

Raum-Zeit-Ausbeute (bezogen auf 3,86 l Reaktionsraum): 49,6 g Roh-1,2-Dichlorethan · h⁻¹ · l⁻¹.

Mittlere Verweilzeit der Gase im Reaktionsraum: 35,3 s.

Analysen des Roh-1,2-Dichlorethans sowie des Abgases siehe nachfolgende Tabellen I und II.

Beispiel 4

Es wird die gleiche Apparatur verwendet wie in Beispiel 1, mit dem nach Art und Menge gleichen Katalysator.

In den Reaktionsraum werden folgende Gasmengen eingespeist:

Luft: 90 Nl · h$^{-1}$;
C$_2$H$_4$: 27 Nl · h$^{-1}$;
HCl: 44 Nl · h$^{-1}$;
Cl$_2$: 4 Nl · h$^{-1}$.

Die Temperatur im Reaktionsrohr wird auf 220°C konstant gehalten, die Versuchsdauer beträgt 3 Stunden.

Folgende Werte werden ermittelt:

Molverhältnis: HCl : C$_2$H$_4$ : Cl$_2$ : O$_2$ = 2 : 1,23 : 0,18 : 0,86.

Umsatz, bezogen auf: HCl = 95,5%; Cl$_2$ = 100%; C$_2$H$_4$ = 96%.

Raum-Zeit-Ausbeute (bezogen auf 3,86 l Reaktionsraum): 28,6 g Roh-1,2-Dichlorethan · h$^{-1}$ · l$^{-1}$.

Mittlere Verweilzeit der Gase im Reaktionsraum: 44,7 s.

Roh-1,2-Dichlorethan- und Abgas-Analysen siehe nachfolgende Tabellen I und II.

**Beispiel 5**

Wiederum wird die gleiche Apparatur und nach Art und Menge der gleiche Katalysator verwendet wie in Beispiel 1, jedoch mit dem Unterschied, dass das Gaseinleitungsrohr mit dem kugelförmigen Ende in dem Stutzen am Reaktionsrohr angeordnet ist, der näher an der zweiten Fritte liegt, so dass, wie oben bereits erwähnt, die Kugel des Gaseinleitungsrohres von der zweiten Fritte in einer Entfernung von 17% der gesamten Länge des Reaktionsraumes liegt. Der entferntere Stutzen am Reaktionsrohr wird nicht benützt, er ist mit einem Stopfen verschlossen. Durch die untere Öffnung des Reaktionsrohres wird ein Gemisch von 90 Nl · h$^{-1}$ Luft und 22 Nl · h$^{-1}$ Chlor eingeleitet, das über die erste Fritte in den Reaktionsraum eintritt. Über die zweite Fritte werden 45 Nl · h$^{-1}$ Ethylen und über das Rohr mit dem kugelförmigen Ende 44 Nl · h$^{-1}$ Chlorwasserstoffgas eingeleitet. Die Temperatur im Reaktionsraum wird auf 222°C konstant gehalten, die Versuchsdauer beträgt 3$^1$/$_2$ Stunden. Im übrigen wird weiterverfahren, wie in Beispiel 1 beschrieben.

Folgende Werte werden ermittelt:

Molverhältnis: C$_2$H$_4$ : HCl : Cl$_2$ : O$_2$ = 2 : 1,95 : 0,98 : 0,84.

Umsatz, bezogen auf: HCl = 85%; Cl$_2$ = 100%; C$_2$H$_4$ = 92,5%.

Raum-Zeit-Ausbeute (bezogen auf 3,86 l Reaktionsraum): 46,6 g Roh-1,2-Dichlorethan · h$^{-1}$ · l$^{-1}$.

Mittlere Verweilzeit der Gase im Reaktionsraum: 36,6 s.

Roh-1,2-Dichlorethan- und Abgas-Analysen siehe unterstehende Tabellen I und II.

Bei allen bis jetzt beschriebenen Beispielen (Nr. 1 bis 5) wird bei normalem Atmosphärendruck von 97,3 kPa gearbeitet.

**Beispiel 6**

Die hierfür verwendete Apparatur ist analog der in Beispiel 1 verwendeten aufgebaut, jedoch mit dem Unterschied, dass als Reaktionsraum ein senkrecht stehendes Nickelrohr mit 50 mm innerem Durchmesser verwendet wird, das ähnlich dem Glasrohr der im Beispiel 1 verwendeten Aparatur ausgestattet ist, mit folgenden Unterschieden: Es sind nur drei Temperatur-Messstellen über den Mantel des Reaktionsrohres gleichmässig verteilt vorhanden. Auch am Kopf des Rohres, unmittelbar vor der Verjüngung, ist eine Fritte eingebaut, die zur Druckminderung und zum Zurückhalten mitgerissener Katalysatorteilchen verwendet wird, die in der Glasapparatur hierfür vorgesehene Kugel entfällt. Am Reaktorausgang ist ein Druckminderungsventil angebracht. Das Gaseinleitungsrohr mit kugelförmigem Kopf ist in den Reaktor fest eingebaut, wobei der Abstand von der Kugel bis zur zweiten Fritte (von unten) 56% der Länge des gesamten Reaktionsraumes, gemessen zwischen der untersten und der obersten Fritte im Rohr, beträgt. Im oberen Teil des Rohres ist eine Druckmessvorrichtung angebracht.

Der Inhalt des Reaktionsraumes abzüglich dem Volumen der darin befindlichen Einbauten (Temperierschlange, Gaseinleitungsrohre mit Fritte bzw. Kugelkopf, Temperaturmessfühler) beträgt 1500 cm$^3$. Am Reaktorausgang nach dem Druckminderventil sind Wasser- und Solekühler sowie Chlorwasserstoff-Wäscher angeschlossen, wie in Beispiel 1 beschrieben.

Das Nickelrohr wird mit 1,2 l (Schüttvolumen) desselben Katalysators, wie in Beispiel 1 beschrieben, gefüllt. Diese Katalysatormenge hat ein Eigenvolumen von 360 cm$^3$. Der für die Gasreaktion zur Verfügung stehende freie Raum beträgt 1140 cm$^3$.

Durch die unterste Gaszuführungsleitung werden zunächst 60 Nl · h$^{-1}$ Luft durch die erste (unterste) Fritte unter Druck geblasen und der Reaktor vermittels der Temperierschlange auf 190°C aufgeheizt. Durch Regulierung des Druckminderventiles am Kopf des Reaktors wird im Reaktor ein Druck von 392 kPa eingestellt. Nach einer halben Stunde ist Temperatur und Druckkonstanz im Reaktionsrohr erreicht. Nun wird die eingeblasene Luftmenge auf 90 Nl · h$^{-1}$ erhöht und durch die zweite Fritte ein Gemisch von 45 Nl · h$^{-1}$ Ethylen und 44 Nl · h$^{-1}$ Chlorwasserstoff eingeblasen. Unmittelbar danach wird durch das Gaseinleitungsrohr mit Kugelkopf dem Reaktor 22 Nl · h$^{-1}$ Chlor zugeführt. Die Temperatur im Reaktionsraum steigt an und wird durch Einführung eines Kühlmediums in die Temperierschlange auf 220°C eingestellt und während weiterer 6 Stunden konstant gehalten. Die in den Reaktionsraum eingeführten Gase werden auf 60°C vorgeheizt. Nach Ablauf der 6 Stunden wird die Gaszufuhr unterbrochen und der Katalysator im Reaktionsrohr mit Luft (etwa Zimmertemperatur) kaltgeblasen.

Während der gesamten Versuchsdauer ist der Wasserkühler mit Wasser von +13°C und der Solekühler mit Kühlsole von −20°C durchflossen. Das Abgas, das den Solekühler verlässt, hat eine Temperatur von +11°C.

Nach 1/3 und 2/3 der gesamten Versuchsdauer werden Abgasproben nach der Chlorwasserstoffwäsche gaschromatographisch untersucht. Der Mittelwert der festgestellten Ergebnisse ist in nachfolgender Tabelle II aufgeführt.

Nach Versuchsende werden die vom Wasser- bzw. Solekühler kondensierten Flüssigkeiten vereinigt gewogen und anschliessend gaschromatographisch analysiert. Das Analysenergebnis ist in nachstehender Tabelle I aufgeführt.

Folgende Werte werden ermittelt:

Molverhältnis: $HCl : C_2H_4 : Cl_2 : O_2 = 2 : 2,05 : 1 : 0,86$.

Umsatz, bezogen auf: $HCl = 99\%$; $Cl_2 = 100\%$; $C_2H_4 = 98,5\%$.

Raum-Zeit-Ausbeute (bezogen auf 1,14 l Reaktionsraum): 169,7 g Roh-1,2-Dichlorethan $\cdot$ h$^{-1}$ $\cdot$ l$^{-1}$.

Mittlere Verweilzeit im Reaktionsraum: 43,7 s.
Analysenergebnisse siehe Tabellen I und II.

Beispiel 7

Es wird die Apparatur, wie in Beispiel 1 beschrieben, verwendet. In das Reaktionsrohr werden 2,8 l (Schüttvolumen) des in Beispiel 1 beschriebenen Katalysators eingefüllt, der jedoch vor seinem Einsatz mit Eisen(III)-chlorid behandelt wurde, so dass er neben 3,7% Kupfer 0,51% Eisen enthält. Das Eigenvolumen der Katalysatorteilchen beträgt 300 cm³.

Nach Erwärmen des Reaktionsrohres auf 185°C, wie in Beispiel 1 beschrieben, werden durch das unterste Gaseinleitungsrohr 90 Nl $\cdot$ h$^{-1}$ Luft durch die erste (unterste) Fritte und ein Gasgemisch von 45 Nl $\cdot$ h$^{-1}$ Ethylen und 44 Nl $\cdot$ h$^{-1}$ Chlorwasserstoff über die zweite Fritte in den Reaktionsraum eingeleitet. Über das Gaseinleitungsrohr mit Kugelkopf werden 22 Nl $\cdot$ h$^{-1}$ Chlor in den Reaktionsraum eingeführt. Vermittels einer Kühlflüssigkeit, die die Temperierschlange durchströmt, wird die Temperatur im Reaktionsraum auf 230°C eingestellt und während 5 Stunden auf dieser Höhe konstant gehalten. Danach wird die Gaszufuhr unterbrochen und der Katalysator im Reaktionsraum mit Luft von Zimmertemperatur kaltgeblasen.

Analysen und Versuchsauswertung erfolgten wie in Beispiel 1 beschrieben.

Es werden folgende Werte ermittelt:

Molverhältnis: $HCl : C_2H_4 : Cl_2 : O_2 = 2 : 2,05 : 1 : 0,86$.

Umsatz, bezogen auf: $HCl = 92\%$; $Cl_2 = 99,9\%$; $C_2H_4 = 95\%$.

Raum-Zeit-Ausbeute (bezogen auf 3,86 l Reaktionsraum): 47,8 g Roh-1,2-Dichlorethan $\cdot$ h$^{-1}$ $\cdot$ l$^{-1}$.

Mittlere Verweilzeit im Reaktionsraum: 36,0 s.

Analysenergebnisse siehe Tabellen I bzw. II.

Beispiel 8

Es wird dieselbe Apparatur verwendet wie in Beispiel 5 und nach Art und Menge derselbe Katalysator wie in diesem Beispiel. Nach Erwärmen des Reaktionsrohrs, wie in Beispiel 1 beschrieben, werden in die unterste Öffnung des Reaktionsrohrs eine Mischung von 72 Nl $\cdot$ h$^{-1}$ Luft und 22 Nl $\cdot$ h$^{-1}$ Chlor durch die erste (unterste) Fritte und 37 Nl $\cdot$ h$^{-1}$ Ethylen durch die zweite Fritte in das Reaktionsrohr eingeleitet, unmittelbar danach werden 30 Nl $\cdot$ h$^{-1}$ Chlorwasserstoff durch das Gaseinleitungsrohr, dessen kugelförmiger Kopf sich in einem Abstand von 17% der gesamten Reaktionsraumlänge von der zweiten Fritte befindet, in den Reaktionsraum eingeführt. Die Temperatur im Reaktionsraum wird auf 208°C während der Versuchsdauer von 2,5 Stunden konstant gehalten. Danach wird die Gaszufuhr unterbrochen und, wie oben mehrfach beschrieben, mit Luft kaltgeblasen.

Folgende Werte werden ermittelt:

Molverhältnis: $C_2H_4 : Cl_2 : HCl : O_2 = 2 : 1,19 : 1,62 : 0,82$.

Umsatz, bezogen auf: $HCl = 85\%$; $Cl_2 = 100\%$; $C_2H_4 = 95\%$.

Raum-Zeit-Ausbeute (bezogen auf 3,86 l Reaktionsraum): 40,2 g Roh-1,2-Dichlorethan $\cdot$ h$^{-1}$ $\cdot$ l$^{-1}$.

Verweilzeit im Reaktionsraum: 46,1 s.

Roh-1,2-Dichlorethan- und Abgas-Analysen siehe nachstehende Tabellen I und II.

Beispiel 9

Es wird verfahren wie in Beispiel 8, jedoch wird die Temperatur im Reaktionsraum auf einem erheblich höheren Niveau, nämlich 246°C während der Versuchsdauer von 3 Stunden konstant gehalten.

Folgende Werte werden ermittelt:

Molverhältnis: $C_2H_4 : Cl_2 : HCl : O_2 = 2 : 1,19 : 1,62 : 0,82$.

Umsatz, bezogen auf: $HCl = 89\%$; $Cl_2 = 100\%$; $C_2H_4 = 96\%$.

Raum-Zeit-Ausbeute (bezogen auf 3,86 l Reaktionsraum): 40,7 g Roh-1,2-Dichlorethan $\cdot$ h$^{-1}$ $\cdot$ l$^{-1}$.

Verweilzeit im Reaktionsraum: 43,6 s.

Die Roh-1,2-Dichlorethan- und Abgas-Analysen sind nachfolgenden Tabellen I bzw. II zu entnehmen.

Die Beispiele 7 bis 9 werden bei normalem Atmosphärendruck, das heisst bei 97,3 kPa durchgeführt.

Tabelle I

Gaschromatographische Analyse des kondensierten Roh-1,2-Dichlorethan

| Komponenten | Beispiel Nr. 1 Gew.-% | Beispiel Nr. 2 Gew.-% | Beispiel Nr. 3 Gew.-% | Beispiel Nr. 4 Gew.-% | Beispiel Nr. 5 Gew.-% | Beispiel Nr. 6 Gew.-% | Beispiel Nr. 7 Gew.-% | Beispiel Nr. 8 Gew.-% | Beispiel Nr. 9 Gew.-% |
|---|---|---|---|---|---|---|---|---|---|
| 1,2-Dichlorethan | 98,527 | 98,996 | 97,814 | 99,132 | 98,892 | 98,494 | 98,458 | 99,363 | 97,598 |
| Summe: $C_2H_2$, $C_2H_4$, $C_2H_5$; | 0,019 | 0,003 | 0,001 | 0,010 | 0,008 | 0,001 | 0,001 | 0,013 | 0,003 |
| Vinylchlorid | 0,009 | 0,007 | 0,009 | 0,010 | 0,005 | 0,007 | 0,021 | 0,006 | 0,007 |
| $C_2H_5Cl$ | 0,030 | 0,029 | | 0,065 | 0,066 | 0,068 | 0,034 | 0,032 | 0,037 |
| 1,2-Dichlorethylen trans. | 0,009 | 0,003 | 0,015 | 0,013 | 0,004 | 0,008 | 0,028 | 0,002 | 0,014 |
| 1,1-Dichlorethan | 0,005 | 0,004 | 0,007 | 0,005 | 0,005 | 0,005 | 0,007 | 0,005 | 0,006 |
| $CCl_4$ | 0,045 | 0,033 | 0,017 | 0,011 | 0,015 | 0,021 | 0,073 | 0,017 | 0,061 |
| 1,2-Dichlorethylen cis | 0,042 | 0,021 | 0,062 | 0,058 | 0,022 | 0,038 | 0,100 | 0,012 | 0,060 |
| $CHCl_3$ | 0,024 | 0,017 | 0,009 | 0,037 | 0,007 | 0,005 | 0,044 | 0,005 | 0,033 |
| 1,1,2-Trichlorethylen | 0,002 | 0,002 | 0,003 | 0,008 | 0,001 | 0,002 | 0,006 | 0,008 | 0,004 |
| 1,1,2-Trichlorethan | 0,882 | 0,616 | 1,054 | 0,422 | 0,662 | 0,834 | 0,807 | 0,413 | 1,361 |
| 2-Chlorethanol | 0,013 | 0,003 | 0,003 | 0,003 | 0,001 | 0,035 | 0,027 | 0,001 | 0,004 |
| 1,1,2,2-Tetrachlorethan | 0,270 | 0,163 | 0,563 | 0,084 | 0,134 | 0,250 | 0,211 | 0,041 | 0,669 |
| $\beta,\beta$-Dichlordiethylether | | | | | | 0,004 | 0,003 | | |
| Chloral | 0,112 | 0,083 | 0,178 | 0,131 | 0,175 | 0,203 | 0,130 | 0,090 | 0,127 |

Tabelle II

Gaschromatographische Analyse des Abgases nach der Chlorwasserstoffwäsche

| Komponenten | Beispiel Nr. 1 Vol.-% | Beispiel Nr. 2 Vol.-% | Beispiel Nr. 3 Vol.-% | Beispiel Nr. 4 Vol.-% | Beispiel Nr. 5 Vol.-% | Beispiel Nr. 6 Vol.-% | Beispiel Nr. 7 Vol.-% | Beispiel Nr. 8 Vol.-% | Beispiel Nr. 9 Vol.-% |
|---|---|---|---|---|---|---|---|---|---|
| $O_2$ | 5,0 | 7,5 | 2,9 | 6,6 | 9,2 | 4,8 | 0,5 | 11,7 | 5,6 |
| CO | 2,0 | 1,0 | 2,5 | 1,5 | 0,90 | 1,4 | 3,4 | 0,60 | 2,4 |
| $CO_2$ | 2,1 | 1,5 | 2,8 | 2,2 | 0,86 | 2,4 | 4,4 | 0,57 | 3,0 |
| $C_2H_4$ | 0,44 | 0,98 | 0,35 | 0,60 | 3,0 | 0,25 | 0,13 | 1,9 | 0,15 |
| Vinylchlorid | 0,006 | 0,002 | 0,008 | 0,006 | 0,003 | 0,006 | 0,015 | 0,004 | 0,005 |
| $C_2H_5Cl$ | 0,016 | 0,009 | 0,179 | 0,015 | 0,034 | 0,038 | 0,016 | 0,016 | 0,020 |
| Leichtsieder | 0,007 | 0,004 | 0,013 | 0,006 | 0,003 | 0,021 | 0,020 | 0,001 | 0,011 |
| 1,2-Dichlorethan (EDC) | 2,4 | 2,0 | 2,15 | 2,4 | 2,4 | 2,4 | 2,2 | 2,8 | 2,8 |
| Hochsieder | 0,0003 | 0,0005 | — | — | <0,001 | <0,001 | 0,010 | <0,001 | <0,001 |
| 1,1,2-Trichlorethan | 0,005 | 0,002 | 0,003 | <0,001 | 0,003 | 0,003 | 0,003 | 0,001 | 0,003 |
| $Cl_2$ im Abgas | Spuren | <2 mg/h | frei | frei | frei | frei | Spuren | frei | frei |
| $Cl_2$ im Wasser | Spuren | 2 ppm | frei | frei | frei | frei | 3 ppm | frei | frei |
| $Cl_2$ im Roh-EDC | frei | Spuren | frei | frei | frei | frei | Spuren | frei | frei |

## Patentansprüche

1. Verfahren zur Herstellung von 1,2-Dichlorethan aus Ethylen durch Umsetzung mit Chlorwasserstoff und Sauerstoff enthaltenden Inertgasen bei 180 bis 300°C und 0,09 bis 1,1 MPa sowie durch Umsetzung mit Chlor in der Gasphase in Gegenwart eines Kupfersalz oder Kupfer- und Eisensalz enthaltenden festen Katalysators; Abkühlung und destillative Auftrennung des Reaktionsgemisches, dadurch gekennzeichnet, dass beide Chlorierungs-Reaktionen in einem gemeinsamen Reaktionsraum, der aufgewirbelte Katalysatorteilchen enthält, nacheinander durchgeführt werden, wobei für die erste Chlorierungs-Reaktion ein stöchiometrischer Überschuss von Ethylen, bezogen auf das Chlorierungsmittel eingesetzt wird und die im gesamten Reaktionsraum gebildete Wärme durch indirekte Kühlung mit einem flüssigen und/oder gasförmigen Wärmeübertragungsmedium abgeführt wird.

2. Verfahren nach Anspruch 1, dadurch gekennzeichnet, dass in dem Reaktionsraum zuerst Chlorwasserstoff, Ethylen und Sauerstoff enthaltendes Inertgas und danach Chlor in folgenden Molverhältnissen eingeführt werden: 2 Mol HCl; 1,01 bis 3 Mol $C_2H_4$; mindestens 0,5 Mol $O_2$ und 0,009 bis 2 Mol $Cl_2$, wobei die Chlormenge so bemessen wird, dass im Gasgemisch, das den Reaktionsraum verlässt, weniger als 0,001 Gew.-% freies, elementares Chlor gefunden werden.

3. Verfahren nach Anspruch 2, dadurch gekennzeichnet, dass die umzusetzenden Stoffe in den Reaktionsraum in folgenden Molverhältnissen eingeführt werden: 2 Mol HCl; 1,8 bis 2,2 Mol $C_2H_4$; 0,5 bis 0,6 Mol $O_2$ und 0,79 bis 1,2 Mol $Cl_2$.

4. Verfahren nach Ansprüchen 2 und 3, dadurch gekennzeichnet, dass an einem Ende eines rohrförmigen Reaktors Ethylen, Chlorwasserstoff und sauerstoffhaltiges Inertgas getrennt oder zumindest teilweise getrennt voneinander eingeleitet werden, und dass in einem Abstand von 40 bis 85% des gesamten Reaktionsraumes von der in Gasströmungsrichtung letzten der genannten Gaseinleitungen entfernt Chlor eingeleitet wird, wobei die Reaktionsprodukte am anderen Ende des Reaktors abgeführt werden.

5. Verfahren nach Ansprüchen 2 bis 4, dadurch gekennzeichnet, dass das Wärmeübertragungsmedium in der indirekten Kühlung im Gegenstrom zu den Gasen im Reaktionsraum geführt wird.

6. Verfahren nach Anspruch 1, dadurch gekennzeichnet, dass in den Reaktionsraum zuerst Ethylen, Chlor und Inertgas, das gegebenenfalls Sauerstoff enthalten kann, und danach Chlorwasserstoff und gegebenenfalls Sauerstoff und Inertgas in folgenden Molverhältnissen eingeführt werden: 2 Mol $C_2H_4$; 0,9 bis 1,2 Mol $Cl_2$; 1,6 bis 2,3 Mol HCl und 0,35 bis 1,3 Mol Gesamt-$O_2$, wobei die Sauerstoff- oder die Chlorwasserstoffmenge so bemessen wird, dass im Gasgemisch, das den Reaktionsraum verlässt, weniger als 0,001 Gew.-% freies, elementares Chlor gefunden werden.

7. Verfahren nach Anspruch 6, dadurch gekennzeichnet, dass an einem Ende eines rohrförmigen Reaktors Ethylen, Chlor und Inertgas, das gegebenenfalls Sauerstoff enthalten kann, zumindest teilweise getrennt voneinander eingeleitet werden, und dass in einem Abstand von 10 bis 40% des gesamten Reaktionsraumes von der in Gasströmungsrichtung letzten der genannten Gaseinleitungen entfernt Chlorwasserstoff und gegebenenfalls Sauerstoff und Inertgas getrennt oder zumindest teilweise getrennt voneinander eingeleitet werden, wobei die Reaktionsprodukte am anderen Ende des Reaktors abgeführt werden.

8. Verfahren nach Ansprüchen 6 und 7, dadurch gekennzeichnet, dass das Wärmeübertragungsmedium in der indirekten Kühlung im Gleichstrom zu den Gasen im Reaktionsraum geführt wird.

9. Verfahren nach Ansprüchen 1 bis 8, dadurch gekennzeichnet, dass der Sauerstoff zu mindestens 50% seiner Gesamtmenge als Luft eingeführt wird.

10. Verfahren nach Ansprüchen 1 bis 9, dadurch gekennzeichnet, dass bei Temperaturen des Reaktionsgemisches von 190 bis 250°C, vorzugsweise von 200 bis 230°C, gearbeitet wird.

11. Verfahren nach Ansprüchen 1 bis 10, dadurch gekennzeichnet, dass bei Drücken im Reaktionsraum von 0,3 bis 0,7 MPa gearbeitet wird.

## Claims

1. Process for the manufacture of 1,2-dichloroethane from ethylene by reaction with hydrogen chloride and inert gases containing oxygen, at 180 to 300°C and 0.09 to 1.1 MPa, and also by reaction with chlorine in the gas phase in the presence of a solid catalyst containing a copper salt or a copper salt and an iron salt; cooling and separating the reaction mixture by distillation, characterised in that the two chlorination reactions are carried out successively in a common reaction space, which contains fluidized catalyst particles, a stoichiometric excess of ethylene, relative to the chlorinating agent, being employed for the first chlorination reaction and the heat formed in the whole reaction space being removed by indirect cooling using a liquid and/or gaseous heat transfer medium.

2. Process according to claim 1, characterised in that hydrogen chloride, ethylene and an inert gas containing oxygen are first introduced into the reaction space, followed by chlorine, in the following molar proportions: 2 moles of HCl; 1.01 to 3 moles of $C_2H_4$; at least 0.5 mole of $O_2$ and 0.009 to 2 moles of $Cl_2$, the quantity of chlorine being such that less than 0.001% by weight of free elementary chlorine is found in the mixture of gases leaving the reaction space.

3. Process according to claim 2, characterised in that the substances to be reacted in the reaction space are introduced in the following molar rations: 2 moles of HCl; 1.8 to 2.2 moles of $C_2H_4$; 0.5 to 0.6 moles of $O_2$ and 0.79 to 1.2 moles of $Cl_2$.

4. Process according to either of claims 2 and 3, characterised in that ethylene, hydrogen chloride, and an inert gas containing oxygen are introduced separately, or at least partially separated from one another, at one end of a tubular reactor, and chlorine is introduced at a distance of 40 to 85% of the total reaction space from the last of the gas inlets mentioned, in the direction of flow of the gas, the reaction products being removed at the other end of the reactor.

5. Process according to any of claims 2 to 4, characterised in that the heat transfer medium in the indirect cooling system is passed in countercurrent to the gases in the reaction space.

6. Process according to claim 1, characterised in that ethylene, chlorine and an inert gas which can optionally contain oxygen are first introduced into the reaction space, followed by hydrogen chloride and, if appropriate, oxygen and an inert gas, in the following molar proportions: 2 moles of $C_2H_4$; 0.9 to 1.2 moles of $Cl_2$; 1.6 to 2.3 moles of HCl and a total of 0.35 to 1.3 moles of $O_2$, the quantity of oxygen or of hydrogen chloride being such that less than 0.001% by weight of free elementary chlorine is found in the mixture of gases leaving the reaction space.

7. Process according to claim 6, characterised in that ethylene, chlorine and an inert gas which can optionally contain oxygen are introduced, at least partially separated from one another, at one end of a tubular reactor, and hydrogen chloride and, if appropriate, oxygen and an inert gas are introduced, seperately, or at least partially seperated from one another, at a distance of 10 to 40% of the total reaction space from the last of the gas inlets mentioned, in the direction of flow of the gas, the reaction products being removed at the other end of the reactor.

8. Process according to either of claims 6 and 7, characterised in that the heat transfer medium in the indirect cooling system is passed in co-current to the gases in the reaction space.

9. Process according to any one of claims 1 to 8, characterised in that the oxygen is introduced in the form of air to the extent of at least 50% of its total quantity.

10. Process according to any one of claims 1 to 9, characterised in that the process is carried out at temperatures, of the reaction mixture, of 190 to 250°C, preferably 200 to 230°C.

11. Process according to any one of claims 1 to 10, characterised in that the process is carried out at pressures, in the reaction space, of 0.3 to 0.7 MPa.

**Revendications**

1. Procédé de production de 1,2-dichlorétha-
ne à partir d'éthylène par réaction avec du chlorure d'hydrogène et de l'oxygène contenu dans un gaz inerte entre 180 et 360°C et sous des pressions de 0,09 à 1,1 MPa, ainsi que par réaction avec du chlore en phase gazeuse, en présence d'un catalyseur solide comprenant un sel de cuivre ou un sel de cuivre et un sel de fer, puis refroidissement et séparation par distillation du mélange réactionnel, procédé caractérisé en ce que les deux réactions de chloration sont effectuées successivement dans une chambre réactionnelle commune contenant un catalyseur divisé en lit tourbillonnaire (lit fluidisé), avec pour la première chloration un excès stoechiométrique d'éthylène par rapport à l'agent de chloration, et la chaleur formée dans la totalité de la chambre réactionnelle est évacuée par un refroidissement indirect avec un milieu caloporteur liquide et/ou gazeux.

2. Procédé selon la revendication 1, caractérisé en ce que l'on fait arriver à la chambre de réaction d'abord le chlorure d'hydrogène, l'éthylène et le gaz inerte contenant l'oxygène, puis le chlore, dans les proportions molaires suivantes: 2 moles d'HCl; 1,01 à 3 moles de $C_2H_4$; au moins 0,5 mole de $O_2$ et 0,009 à 2 moles de $Cl_2$, la quantité de chlore étant déterminée de manière que dans le mélange gazeux sortant du réacteur on trouve moins de 0,001% en poids de chlore élémentaire libre.

3. Procédé selon la revendication 2, caractérisé en ce que les réactifs sont introduits dans la chambre dans les proportions molaires de 2 moles d'HCl; 1,8 à 2,2 moles de $C_2H_4$; 0,5 à 0,6 mole de $O_2$ et 0,79 à 1,2 mole de $Cl_2$.

4. Procédé selon la revendication 2 ou 3, caractérisé en ce que l'on fait arriver par une extrémité d'un réacteur tubulaire l'éthylène, le chlorure d'hydrogène et le gaz inerte contenant l'oxygène, séparément les uns des autres ou tout au moins en partie séparément, et le chlore à une distance du dernier des conduits d'arrivée de gaz, dans le sens de circulation des gaz, qui représente 40 à 85% de la longueur totale de la chambre de réaction, les produits de la réaction étant évacués à l'autre extrémité du réacteur.

5. Procédé selon l'une quelconque des revendications 2 à 4, caractérisé en ce que le milieu caloporteur de refroidissement circule à contre-courant des gaz.

6. Procédé selon la revendication 1, caractérisé en ce que l'on fait arriver à la chambre de réaction d'abord l'éthylène, le chlore et le ganz inerte pouvant éventuellement contenir de l'oxygène, puis le chlorure d'hydrogène avec éventuellement de l'oxygène et du gaz inerte, dans les proportions molaires: 2 moles de $C_2H_4$; 0,9 à 1,2 mole de $Cl_2$; 1,6 à 2,3 moles d'HCl et 0,35 à 1,3 mole de $O_2$ total, la quantité d'oxygène ou de chlorure d'hydrogène étant déterminée de manière que dans le mélange gazeux qui sort du réacteur on trouve moins de 0,001% en poids de chlore élémentaire libre.

7. Procédé selon la revendication 6, caractérisé en ce que l'on fait arriver par une extrémité d'un réacteur tubulaire l'éthylène, le chlore et le gaz inerte pouvant contenir éventuellement de l'oxygène, au moins en partie séparément les uns des autres, et à une distance du dernier des conduits d'arrivée de gaz représentant 10 à 40% de la longueur totale du réacteur, dans le sens de circulation des gaz, on fait arriver le chlorure d'hydrogène, avec éventuellement de l'oxygène et du gaz inerte, séparément les uns des autres ou au moins en partie séparément, les produits de la réaction étant évacués à l'autre extrémité du réacteur.

8. Procédé selon la revendication 6 ou 7, caractérisé en ce que le milieu caloporteur de refroidissement circule dans le même sens que les gaz en réaction.

9. Procédé selon l'une quelconque des revendications 1 à 8, caractérisé en ce que l'oxygène, pour au moins 50% de sa quantité totale, est introduit sous forme d'air.

10. Procédé selon l'une quelconque des revendications 1 à 9, caractérisé en ce que l'on opère à des températures du mélange réactionnel de 190 à 250°C, de préférence de 200 à 230°C.

11. Procédé selon l'une quelconque des revendications 1 à 10, caractérisé en ce que l'on opère sous des pressions dans la chambre de réaction de 0,3 à 0,7 MPa.